# Europäisches Patentamt
## European Patent Office
## Office européen des brevets

(11) Veröffentlichungsnummer: **0 308 362 B1**

(12) **EUROPÄISCHE PATENTSCHRIFT**

(45) Veröffentlichungstag der Patentschrift: **25.05.94**

(51) Int. Cl.5: **C07C 59/265**, C07C 51/41, //A61K31/19

(21) Anmeldenummer: **88730208.1**

(22) Anmeldetag: **09.09.88**

Die Akte enthält technische Angaben, die nach dem Eingang der Anmeldung eingereicht wurden und die nicht in dieser Patentschrift enthalten sind.

(54) **Eisen-Citrat-Komplex, Verfahren für seine Herstellung und seine pharmazeutische Verwendung.**

(30) Priorität: **14.09.87 IT 2190487**

(43) Veröffentlichungstag der Anmeldung:
**22.03.89 Patentblatt 89/12**

(45) Bekanntmachung des Hinweises auf die Patenterteilung:
**25.05.94 Patentblatt 94/21**

(84) Benannte Vertragsstaaten:
**AT BE CH DE ES FR GB GR IT LI LU NL SE**

(56) Entgegenhaltungen:
**JP-A- 1 134 158**
**US-A- 4 400 535**

**PATENT ABSTRACTS OF JAPAN, Band 8, Nr. 3 (C-203)[1440], 7. Januar 1984; & JP-A-58 172 343**

(73) Patentinhaber: **SCHERING AKTIENGESELL-SCHAFT**

**D-13342 Berlin(DE)**

(72) Erfinder: **Antonini, Eraldo**
**c/o Pettini**
**Via Parione 37**
**I-00186 Rom(IT)**
Erfinder: **Vidic, Hans-Jörg**
**Linderthaler Allee 8**
**D-1000 Berlin 37(DE)**

## Beschreibung

Die vorliegende Erfindung betrifft einen neuen Eisen-Citrat-Komplex, Verfahren für seine Herstellung und seine pharmazeutische Verwendung.

Für die Therapie der Eisenmangelanämie werden seit langem Eisenverbindungen mit zwei- und dreiwertigem Eisen sowie Eisenchelate mit niedrigem oder höherem Molekulargewicht verwendet. Die meisten dieser Eisenpräparate führen bei längerer Anwendung zu gastro-intestinalen Komplikationen oder sind von gewisser Toxizität. Ferritin hat sich dagegen als ein lokal wie allgemein verträgliches Mittel erwiesen. Der Nachteil des Ferritins ist jedoch, daß es nur in begrenzter Menge zur Verfügung steht, weil es außer Pferdemilz keine nennenswerten Rohstoffquellen für Ferritin gibt.

Aufgabe der Erfindung war es deshalb, eine Eisenverbindung bereitzustellen, deren Micellar-Struktur der Struktur der Micellen des Ferritinkerns sehr ähnlich ist, ohne daß dabei die günstigen pharmazeutischen und toxikologischen Eigenschaften des Ferritins verlorengehen.

Aus der japanischen Auslegeschrift JP 18 798/61 ist ein Verfahren zur Herstellung eines wasserlöslichen Eisen-Komplexes aus Alkalicitrat und Eisencarbonat bekannt. Das aus 1 Mol $FeCl_3 \cdot 6H_2O$ und 1,5 Mol Natriumbicarbonat bei Temperaturen unterhalb 13° C gebildete Eisencarbonat wird mit Natriumcitrat zu einem aus rund 60 % Eisen bestehenden Eisenkomplexsalz umgesetzt.

Es wurde nun überraschend gefunden, daß aus $FeCl_3 \cdot 6H_2O$, $NaHCO_3$ und Trinatriumcitrat ein braungefärbter Eisen-Citrat-Micellen-Komplex entsteht, der sich durch folgende Eigenschaften auszeichnet:

a) Zusammensetzung: 10,6 % Kohlenstoff, 2,62 % Wasserstoff, 50,2 % Sauerstoff, 32 % Eisen, 4,6 % Natrium;

b) Molekulargewicht etwa 33.000 (HPLC-LALLS Messung);

c) empirische Formel $(C_{31}H_{92}O_{110}Fe_{20}Na_7)_n$ mit n = 9-10;

d) löslich in Wasser, Glycerin-Wasser-Gemischen, praktisch unlöslich in üblichen organischen Lösungsmitteln;

e) Ultraviolettabsorption in Wasser: $\lambda$ max.(470 nm)

$$E_{1}^{1}\frac{\%}{cm} = 23 - 26,5;$$

f) Widerstand (1%ige Lösung) 600 - 750 $\Omega \cdot cm$; Dieser neue Eisen-Citrat-Komplex ist auch in niedrigen pH-Bereichen stabil. Er besitzt damit den Vorteil, als Wirkstoff den Magen ohne Zersetzung zu passieren. Die Zusammensetzung des neuen Komplexes schwankt in dem für Elementaranalysen üblichen Bereich. Die angegebene Zusammensetzung stellt daher nur einen Mittelwert dar, auf den der neue Komplex nicht begrenzt sein soll.

Der erfindungsgemäße Eisen-Citrat-Komplex zeichnet sich durch außerordentliche Stabilität aus. So erwiesen sich bei Raumtemperatur gelagerte Lösungen des neuen Komplexes in einem Wasser/Glycerin-Gemisch (40 : 60) noch nach 3 Jahren als stabil. Sie zeigten keinerlei Anzeichen von Zersetzung, d. h. es trat z. B. keine Ausflockung auf.

Der neue Komplex erweist sich in allen Untersuchungan als weitgehend einheitliche polymere Verbindung. So konnte im Elektropherogramm einer Lösung dieses Komplexes im Puffer auf Celluloseacetatfolie stets nur eine einheitliche Bande nachgewiesen werden.

Weitere Analysen ergaben für den Komplex einen Gehalt an gebundenem Citrat von ca. 28 %.

Die Erfindung betrifft auch ein Verfahren zur Herstellung des Eisen-Citrat-Micellen-Komplexes mit den oben genannten Eigenschaften, das dadurch gekennzeichnet ist, daß man zu einer wäßrigen Lösung von $FeCl_3 \cdot 6H_2O$ festes $NaHCO_3$ zugibt, das freigesetzte $CO_2$ aus der Lösung mit einem Inertgas oder durch Vakuumentgasung entfernt, festes Trinatriumcitrat hinzufügt und aus der erhaltenen Lösung nach längerem Äquilibrieren bei Raumtemperatur das Verfahrensprodukt mit Methanol ausfällt.

Statt durch Methanolfallung läßt sich das Verfahrensprodukt auch durch Dialyse oder Gelfiltration und anschließende Gefriertrocknung erhalten. Bevorzugt ist jedoch die Methanolfällung.

Die Erfindung betrifft außerdem Arzneimittel, enthaltend den erfindungsgemäßen Eisen-Citrat-Micellen-Komplex und nichttoxische, pharmazeutisch unbedenkliche Streckmittel und Hilfsstoffe. Unter diese Streckmittel und Hilfsstoffe fallen alle dem Fachmann auf diesem Gebiet geläufigen Stoffe und Verbindungen. Der neue Komplex kann auch in Kombination mit Folsäure appliziert werden. Das neue Mittel eignet sich auch zur Behandlung von Erkrankungen, die mit Eisendefiziten einhergehen. Der zur Behandlung von Eisenmangel bei Kindern, Frauen, Rekonvaleszenten, Leistungssportlern eingesetzte Eisen-Citrat-Komplex kann in Form von Lösungen, Kapseln, Dragees und Tabletten appliziert werden.

Der neue Eisen-Citrat-Komplex besitzt im Vergleich zu Ferritin bessere pharmakologische und toxikologische Eigenschaften.

Bei Verabreichung verschiedener Eisenpräparate an männliche Wistar-Ratten (Gewicht 150 - 170 g) steigen die Eisenblutwerte bei Applikation mit dem neuen Komplex stärker an als mit Ferritin oder mit $FeSO_4$ (gemessen als Serum-Eisen in µg% nach 6 Stunden Behandlung).

Der neue Komplex ist selbst bei hohen Dosen von > 800 mg/kg nicht toxisch. Bei Rattenversuchen wurden auch nach 1,3 und 7 Tagen keinerlei Veränderungen der Magenschleimhaut beobachtet.

Beispiel 1

Zu einer 0,3 molaren wäßrigen $FeCl_3$-Lösung (hergestellt aus $FeCl_3 \cdot 6H_2O$ durch Lösen in Wasser) werden unter Rühren 2,5 Aquivalente $NaHCO_3$ zugegeben. Anschließend wird aus der dunkelbraun gefärbten Lösung entweder durch Einleiten eines Inertgases (Stickstoff oder Argon) oder duch Vakuum-Entgasung $CO_2$ entfernt. Sobald der größte Teil des $CO_2$ entfernt ist, wird unter Schütteln eine zum Eisen stöchiometrische Menge an festem Trinatriumcitrat hinzugefügt und die Lösung daraufhin bis zu 20 Stunden bei Raumtemperatur stehengelassen. Die so erhaltene Lösung ist klar, intensiv gefärbt und besitzt einen nahezu neutralen pH-Wert.

Danach wird die Lösung gegen Wasser oder Phosphatpuffer (pH 7, 0,1 - 0,01 molar) dialysiert, um alle Bestandteile mit einem niedrigen Molekulargewicht zu entfernen. Als Maß für den Fortgang der Dialyse dient die Leitfähigkeit des Dialysats, die bei Einsatz der nicht eingeengten Lösung einen Wert von ca. 700 $\Omega \cdot cm$ haben soll. Dieser Verfahrensschritt der Entsalzung kann auch mittels einer Gelfiltration über eine Säule mit Sephadex G 25 (Pharmacia) in 2,5%igem Citrat-Puffer erfolgen. Die Lösung wurde bis zu einem Wassergehalt von etwa 2 % gefriergetrocknet. Ausbeute an Eisenmicellen bezogen auf eingesetztes Fe(III)-chlorid beträgt 31 %.

Die Ausbeute bezogen auf Eisen ( = korrigierte theoretische Ausbeute) 66 %.
Elementaranalyse: C 10,65 %; H 2,66 %; Na 4,5 %; Fe 32,76 %.
Extinktion (0,1%ige Lösung bei 470 nm): 1,06.
pH (1%ige Lösung): 7,8.
spezifische Extinktion (bezogen auf 1 % Eisengehalt): 23,9.
pH, bei dem Trübung eintritt (1%ige Lösung): 2,9.
Wassergehalt (nach K. Fischer in %): 2,1.

Beispiel 2

Eine Lösung von 8,1 g (0,03 Mol) $FeCl_3 \cdot 6H_2O$ in 95 ml entionisiertem Wasser wird vorgelegt. Innerhalb 10 Minuten werden 6,3 g (0,075 Mol) Natriumhydrogencarbonat in fester Form, unter Rühren, zugegeben. Dabei tritt starke Gasenwicklung ($CO_2$) auf und das pH steigt auf 2,5.

Zum Entfernen des Kohlendioxyds wird ca. 1 Stunde Stickstoff durch die Lösung geblasen. Danach erfolgt Zugabe von 8,8 g (0,03 Mol) Trinatriumcitrat $\cdot 2H_2O$ in einer Portion unter Rühren. Die auftretende Fällung löst sich nach ca. 5 Minuten wieder auf, wobei das pH auf 5,7 ansteigt. Nach einer Rührzeit von 30 Minuten kann ein pH-Wert von 6,4 gemessen werden. Die Lösung wird ohne Rühren ca. 15 Stunden stehen gelassen. Während dieser Zeit erhöht sich das pH auf ca. 7,9.

Die erhaltene Lösung (110 ml) wird unter Rühren mit 88 ml Methanol versetzt. Die Fällung wird 30 Minuten nachgerührt und danach über eine Nutsche abgesaugt. Der Filterkuchen wird mit einem Turbinen-Rührer in 55 ml Wasser/Methanol Gemisch (1 + 2) suspendiert, erneut abgesaugt und mit 15 ml Methanol gewaschen. Anschließend wird der Rückstand bei 50° C getrocknet.

Die Ausbeute beträgt 3,60 g (85 % des eingesetzten Eisens).
Elementaranalyse: C 10,56 %; H 2,60 %; Na 4,8 %; Fe 30,98 %.
Extinktion (0,1%ige Lösung bei 470 nm): 0,912.
pH (1%ige Lösung): 7,9.
Widerstand (1%ige Lösung): 607,5 $\Omega \cdot cm$.
spezifische Extinktion (bezogen auf 1 % Eisengehalt): 25,55.
pH, bei dem Trübung eintritt (1%ige Lösung): 2,66.
Wassergehalt (nach K. Fischer in %): 6,15.

**Patentansprüche**
**Patentansprüche für folgende Vertragsstaaten : AT, BE, CH, DE, FR, GB, GR, IT, LI, LU, NL, SE**

1. Eisen-Citrat-Micellenkomplex, dadurch gekennzeichnet, daß es ein brauner fester Stoff mit nachfolgend genannten Eigenschaften ist:
   a) Zusammensetzung:
   10,6% Kohlenstoff,
   2,62% Wasserstoff,
   50,2% Sauerstoff,
   32% Eisen,
   4,6% Natrium;
   b) Molekulargewicht etwa 33000 (HPLC-LALLS Messung)
   c) empirische Formel $(C_{31}H_{92}O_{110}Fe_{20}Na_7)_n$ mit n = 9-10;
   d) löslich in Wasser, Glycerin-Wasser-Gemischen, praktisch unlöslich in üblichen organischen Lösungsmitteln;
   e) Ultraviolettabsorption in Wasser: λ max. 470 nm;

   $$E_{1cm}^{1\%} = 23\text{-}26,5;$$

   f) Widerstand (1%ige Lösung) 600-750 $\Omega \cdot cm$.
   erhältlich durch Umsetzung einer wäßrigen Lö-

sung von $FeCl_3 \cdot 6H_2O$ mit festem $NaHCO_3$, Entfernung des freigesetzten $CO_2$ aus der Lösung mit einem Inertgas oder durch Vakuumentgasung, Zusatz von festem Trinatriumcitrat und Fällung aus der erhaltenen Lösung nach längerem Stehen bei Raumtemperatur mit Methanol.

2. Verfahren zur Herstellung des Eisen-Citrat-Micellenkomplexes gemäß Anspruch 1, dadurch gekennzeichnet, daß man zu einer wäßrigen Lösung von $FeCl_3 \cdot 6H_2O$ festes $NaHCO_3$ zugibt,das freigesetzte $CO_2$ aus der Lösung mit einem Inertgas oder durch Vakuumentgasung entfernt, festes Trinatriumcitrat hinzufügt und aus der erhaltenen Lösung nach längerem Stehen bei Raumtemperatur mit Methanol das Verfahrensprodukt ausfällt.

3. Verfahren nach Anspruch 2, dadurch gekennzeichnet, daß man anstelle der Methanolfällung eine Dialyse oder Gelfiltration und Gefiertrocknung durchführt.

4. Arzneimittel, enthalten den Eisen-Citrat-Micellenkomplex gemäß Anspruch 1 und nicht-toxische, pharmazeutisch unbedenkliche Streckmittel und Hilfsstoffe.

**Patentanspruch für folgenden Vertragsstaat : ES**

1. Verfahren zur Herstellung eines braunen und festen Eisen-Citrat-Micellenkomplexes mit folgenden Eigenschaften:
   a) Zusammensetzung:
   10,6 % Kohlenstoff,
   2,62% Wasserstoff,
   50,2% Sauerstoff,
   32 % Eisen,
   4,6 % Natrium;
   b) Molekulargewicht etwa 33000 (HPLC-LALLS Messung)
   c) empirische Formel $(C_{31}H_{92}O_{110}Fe_{20}Na_7)_n$ mit n = 9-10;
   d) löslich in Wasser, Glycerin-Wasser-Gemischen, praktisch unlöslich in üblichen organischen Lösungsmitteln;
   e) Ultraviolettabsorption in Wasser: λ max. 470 nm; $E_{1cm}^{1\%} = 23-26,5$;
   f) Widerstand (1%ige Lösung) 600-750 Ω•cm,
   dadurch gekennzeichnet, daß man zu einer wäßrigen Lösung von $FeCl_3 \cdot 6H_2O$ festes $NaHCO_3$ zugibt, das freigesetzte $CO_2$ aus der Lösung mit einem Inertgas oder durch Vakuumentgasung entfernt, festes Trinatriumcitrat hinzufügt und aus der erhaltenen Lösung nach längerem Äquilibrieren bei Raumtemperatur das Verfahrensprodukt mit Methanol ausfällt.

**Claims**

**Claims for the following Contracting States : AT, BE, CH, DE, FR, GB, GR, IT, LI, LU, NL, SE**

1. Iron citrate micelle complex, characterised in that it is a brown solid substance having the properties mentioned below:
   a) composition:
   10.6% carbon,
   2.62% hydrogen,
   50.2% oxygen,
   32% iron,
   4.6% sodium;
   b) molecular weight approximately 33,000 (HPLC-LALLS measurement);
   c) empirical formula $(C_{31}H_{92}O_{110}Fe_{20}Na_7)_n$ wherein n = 9-10;
   d) soluble in water, glycerine/water mixtures, virtually insoluble in customary organic solvents;
   e) ultraviolet absorption in water: λ max. 470 nm;

$$E_{1cm}^{1\%} = 23-26.5;$$

   f) resistance (1% solution) 600-750 Ω•cm;
   obtainable by reacting an aqueous solution of $FeCl_3 \cdot 6H_2O$ with solid $NaHCO_3$, removing from the solution the $CO_2$ that is liberated with an inert gas or by means of vacuum degassing, adding solid trisodium citrate, and precipitating from the resulting solution with methanol after standing at room temperature for a prolonged period.

2. Process for the preparation of the iron citrate micelle complex according to claim 1, characterised in that solid $NaHCO_3$ is added to an aqueous solution of $FeCl_3 \cdot 6H_2O$, the $CO_2$ that is liberated is removed from the solution with an inert gas or by means of vacuum degassing, solid trisodium citrate is added, and the process product is precipitated from the resulting solution with methanol after standing at room temperature for a prolonged period.

3. Process according to claim 2, characterised in that dialysis or gel filtration and freeze drying are carried out instead of precipitation with methanol.

4. Medicament containing the iron citrate micelle complex according to claim 1 and non-toxic, pharmaceutically acceptable extenders and adjuvants.

**Claim for the following Contracting State : ES**

1. Process for the preparation of a brown solid iron citrate micelle complex having the following properties:

   a) composition:

   10.6% carbon,

   2.62% hydrogen,

   50.2% oxygen,

   32% iron,

   4.6% sodium;

   b) molecular weight approximately 33,000 (HPLC-LALLS measurement);

   c) empirical formula $(C_{31}H_{92}O_{110}Fe_{20}Na_7)_n$ wherein n = 9-10;

   d) soluble in water, glycerine/water mixtures, virtually insoluble in customary organic solvents;

   e) ultraviolet absorption in water: $\lambda$ max. 470 nm;

   $$E_{1cm}^{1\%} = 23\text{--}26.5;$$

   f) resistance (1% solution) 600-750 $\Omega \cdot cm$; characterised in that solid $NaHCO_3$ is added to an aqueous solution of $FeCl_3 \cdot 6H_2O$, the $CO_2$ that is liberated is removed from the solution with an inert gas or by means of vacuum degassing, solid trisodium citrate is added, and the process product is precipitated from the resulting solution with methanol after equilibration at room temperature for a prolonged period.

**Revendications**
**Revendications pour les Etats contractants suivants : AT, BE, CH, DE, FR, GB, GR, IT, LI, LU, NL, SE**

1. Complexe micellaire de citrate de fer caractérisé en ce qu'il est une matière solide, de couleur marron, avec les caractéristiques suivantes :

   a) Composition :

   10,6 % carbone,

   2,62 % hydrogène,

   50,2 % oxygène,

   32 % fer,

   4,6 % sodium ;

   b) poids moléculaire d'environ 33000 (mesuré par HPLC-LALLS)

   c) formule empirique $(C_{31}H_{92}O_{110}Fe_{20}Na_7)_n$ avec n = 9-10 ;

d) soluble dans l'eau, dans des mélanges glycérine-eau, pratiquement insoluble dans les solvants organiques usuels ;

e) absorption de rayons UV dans l'eau : $\lambda$ max. 470 nm

$$E_{1\ cm}^{1\ \%} = 23 - 26,5\ ;$$

f) résistance (solution à 1 %) 600 - 750 $\Omega \cdot cm$ ;

   que l'on peut obtenir par réaction d'une solution aqueuse de $FeCl_3.6H_2O$ avec $NaHCO_3$ solide, par élimination de la solution du $CO_2$ dégagé avec un gaz inerte ou par dégazage sous vide, par addition d'un citrate trisodique solide et par précipitation à partir de la solution obtenue, après repos prolongé à température ambiante, avec le méthanol.

2. Procédé pour la préparation d'un complexe micellaire de citrate de fer selon la revendication 1, caractérisé en ce qu'on ajoute à une solution aqueuse de $FeCl_3 \cdot 6H_2O$ du $NaHCO_3$ solide, en ce qu'on élimine de la solution le $CO_2$ dégagé avec un gaz inerte ou par dégazage sous vide, en ce qu'on ajoute du citrate trisodique solide et en ce que, à partir de la solution obtenue, après un repos prolongé à température ambiante, on précipite le produit du procédé avec du méthanol.

3. Procédé selon la revendication 2, caractérisé en ce qu'on effectue, au lieu de la précipitation avec le méthanol, une dialyse ou une filtration sur gel et une lyophilisation.

4. Médicaments, contenant le complexe micellaire du citrate de fer selon la revendication 1, et des excipients et adjuvants non toxiques, pharmaceutiquement inoffensifs.

**Revendication pour l'Etat contractant suivant : ES**

1. Procédé pour la préparation d'un complexe micellaire du citrate de fer de couleur marron, solide, ayant des caractéristiques suivantes :

   a) Composition :

   10,6 % carbone,

   2,62 % hydrogène,

   50,2 % oxygène,

   32 % fer,

   4,6 % sodium ;

   b) poids moléculaire d'environ 33000 (mesuré par HPLC-LALLS)

c) formule empirique $(C_{31}H_{92}O_{110}Fe_{20}Na_7)_n$ avec n = 9-10 ;

d) soluble dans l'eau, dans des mélanges glycérine-eau, pratiquement insoluble dans les solvants organiques usuels ;

e) absorption de rayons UV dans l'eau : $\lambda$ max. 470 nm

$$E^{1\,\%}_{1\ cm} = 23 - 26,5 \ ;$$

f) résistance (solution à 1 %) 600 - 750 $\Omega \cdot cm$ ;

caractérisé en ce qu'on ajoute à une solution aqueuse de $FeCl_3 \cdot 6H_2O$ du $NaHCO_3$ solide, en ce qu'on élimine de la solution le $CO_2$ dégagé avec un gaz inerte ou par dégazage sous vide, en ce qu'on ajoute du citrate trisodique solide et en ce que, à partir de la solution obtenue, après un repos prolongé à température ambiante, on précipite le produit du procédé avec du méthanol.